Europäisches Patentamt

(19) European Patent Office

Office européen des brevets

(11) Numéro de publication: **0 195 171**
**B1**

(12) **FASCICULE DE BREVET EUROPÉEN**

(45) Date de publication du fascicule du brevet:
**29.03.89**

(51) Int. Cl.⁴: **C 07 C 39/24**, C 07 C 37/62

(21) Numéro de dépôt: **85420222.3**

(22) Date de dépôt: **10.12.85**

(54) **Procédé de préparation de monochlorohydroquinones.**

(30) Priorité: **19.12.84 FR 8419748**

(43) Date de publication de la demande:
**24.09.86 Bulletin 86/39**

(45) Mention de la délivrance du brevet:
**29.03.89 Bulletin 89/13**

(84) Etats contractants désignés:
**AT BE CH DE GB IT LI LU NL SE**

(56) Documents cité:
**FR-A-950 265**
**FR-A-2 187 735**
**US-A-2 748 173**

**CHEMICAL ABSTRACTS, vol. 95, no. 9, 31 août 1981, page 738, no. 80474g, Columbus, Ohio, US**

**Le dossier contient des informations techniques présentées postérieurement au dépôt de la demande et ne figurant pas dans le présent fascicule.**

(73) Titulaire: **RHONE- POULENC CHIMIE, 25, quai Paul Doumer, F-92408 Courbevoie Cédex (FR)**

(72) Inventeur: **Ratton, Serge, Hameau de Belmont Vaulx Milieu, F-38090- Villefontaine (FR)**

(74) Mandataire: **Vignally, Noel, RHONE- POULENC INTERSERVICES Service Brevets Chimie Centre de Recherches de Saint- Fons B.P. 62, F-69192 Saint- Fons Cedex (FR)**

## EP 0 195 171 B1

**Description**

La présente invention a pour objet un procédé de préparation de monohalogénohydroquinones et en particulier de monochlorohydroquinone par halogénation de l'hydroquinone.

Les monohalogénohydroquinones et notamment les monochloro- et monobromohydroquinones sont des produits industriels importants utilisés comme intermédiaires en synthèse organique, dans l'industrie photographique (cf. brevets américains 2 748 173 et 1 912 744) ou comme composés dihydroxylés pour la préparation de polyesters anisotropes (cf. brevet américain No. 4 118 372 et demande de brevet français No. 79-24 135 publiée sous le No. 2 465 758). La monochlorohydroquinone est tout particulièrement recherchée pour ces utilisations.

Bien que diverses voies d'accès aux monohalogénohydroquinones et notamment à la monochlorohydroquinone aient été proposées au départ de composés tels que la p-benzoquinone et l'orthochlorophénol, la méthode la plus intéressante d'un point de vue industriel consiste à procéder à l'halogénation de l'hydroquinone produit courant et bon marché. A cet effet plusieurs procédés ont été décrits. Ainsi dans le brevet américain No. 1 912 744 on a préconisé de préparer les monochloro- et monobromohydroquinones par passage de chlore ou de brome dans une suspension d'hydroquinone dans le tétrachlorure de carbone. Selon le brevet américain 2 748 173 la monochlorohydroquinone est préparée par passage de chlore gazeux dans une solution concentrée d'hydroquinone dans de l'acide acétique aqueux. Ces deux procédés présentent l'inconvénient majeur de conduire, quelles que soient les précautions prises, à la formation concomittante de dichloro-2,3 et/ou -2,5 hydroquinones en quantités d'autant plus importantes que le taux de transformation de l'hydroquinone est plus élevé.

En outre les rendements en monochlorohydroquinone indiqués dans le brevet us 2 748 173 sont calculés indirectement, sans dosage précis et ne tiennent donc pas compte de la formation des sous-produits lourds, que génère la réaction de chloration.

Pour pallier cet inconvénient on a proposé dans la demande japonaise publiée sous le No. 56-45 433 de procéder à la chloration de l'hydroquinone au moyen d'acide chlorhydrique dilué en présence de sels de cuivre ou de fer comme catalyseurs et en opérant sous pression d'oxygène. Bien que ce procédé semble convenir à une production plus sélective de la monochlorohydroquinone son intérêt est diminué par la nécessité de recourir à un appareillage résistant à la pression et à la mise en oeuvre de sels métalliques. Dans les brevets américains 4 439 595 et 4 439 596 on a proposé de remplacer le chlore par le chlorure de sulfuryle et de conduire la chloration dans des esters d'alkyle ou dans l'acide acétique glacial afin d'obtenir des mélanges à haute teneur en monochlorohydroquinone directement utilisables pour la préparation du diacétate correspondant destiné à l'obtention de polyesters anisotropes. Dans ce cas la présence de quantités notables de dichlorohydroquinones et d'hydroquinone non transformée n'est pas préjudiciable au but poursuivi; les rendements en dichlorohydroquinone par rapport à l'hydroquinone transformée reste supérieurs à 10 % et le taux de transformation de l'hydroquinone inférieur à 90 %.

On a encore proposé de procéder à l'halogénation de l'hydroquinone au moyen d'halogènes à l'état naissant formés "in situ" par oxydation d'un hydracide au moyen d'un oxydant. Ainsi dans le brevet anglais 563 541 on a décrit un procédé de préparation de mono- ou polychlorohydroquinones par le chlore naissant formé par addition de bioxyde de manganèse à une suspension d'hydraquinone dans l'acide chlorhydrique concentré. La mise en oeuvre de l'oxyde de manganèse limite l'intérêt industriel de ce procédé parce qu'elle implique la manipulation et l'addition d'un produit solide à la suspension d'hydroquinone dans l'acide chlorhydrique et nécessite une régénération laborieuse de l'oxyde de manganèse à partir du chlorure de manganèse sous-produit. Dans le brevet français No. 73-20 507 publié sous le No. 2 187 735 on a proposé de procéder à l'halogénation de divers composés aromatiques parmi lesquels l'hydroquinone au moyen du couple halogène (chlore, brome, iode)/eau oxygénée afin de récupérer sous forme d'halogène l'hydracide libéré par l'halogénation directe du composé aromatique. Ce procédé met donc en oeuvre une première étape habituelle d'halogénation du substrat par un défaut d'halogène par rapport à la stoechiométrie et une deuxième étape d'halogénation du substrat non transformé au moyen de l'halogène formé "in situ" par oxydation de l'hydracide sous-produit par l'eau oxygénée. Ce procédé est avantageusement conduit dans um système en deux phases comportant une phase aqueuse et une phase organique. On a constaté que dans le cas de la chloration de l'hydroquinone ce procédé ne permet pas de pallier les inconvénients des procédés antérieurs, notamment à l'égard de la formation des polychlorohydroquinones; par ailleurs une quantité importante de l'hydroquinone subit dans ces conditions une oxydation par l'eau oxygénée et/ou le chlore naissant, en particulier lorsque le milieu est hétérogène.

Il s'avère donc qu'en dépit des recherches conduites jusqu'ici il n'existe pas de procédé permettant d'halogéner l'hydroquinone de façon simple, plus sélective et plus complète.

Un premier objet de la présente invention réside dans un procédé d'halogénation de l'hydroquinone en monohalogénohydroquinone qui permet d'abaisser notablement la formation des dihalogénohydroquinones.

Un deuxième objet de la présente invention réside dans un procédé d'halogénation de l'hydroquinone en monohalogénohydroquinone qui permet d'augmenter le taux de transformation de l'hydroquinone et par conséquent la productivité du procédé.

Un troisième objet de la présente invention réside dans un procédé d'halogénation de l'hydroquinone en monohalogénohydroquinone qui permet la mise en oeuvre industrielle plus aisée en raison des réactifs et des conditions de la réaction.

2

Un quatrième objet de la présente invention réside dans un procédé de chloration de l'hydroquinone en monochlorohydroquinone au moyen de chlore à l'état naissant qui permet d'éviter ou de limiter l'oxydation parasite de l'hydroquinone.

Plus spécifiquement la présente invention a pour objet un procédé de chloration d'hydroquinone essentiellement en monochlorohydroquinone au moyen de chlore à l'état naissant caractérisé en ce que l'on fait réagir l'hydroquinone avec une solution aqueuse d'acide chlorhydrique et de l'eau oxygénée en présence d'un solvant organique de l'hydroquinone inerte dans les conditions de la réaction choisi parmi les acides alcanoïques et les étheroxydes aliphatiques ou hétérocycliques.

Comme exemples d'acides alcanoïques on peut citer l'acide acétique, l'acide propionique, l'acide n-pentanoïque, l'acide n-hexanoïque; on utilise de préférence les acides alcanoïques inférieurs et en particulier l'acide acétique. Les étheroxydes constituent une classe préférée de solvants de l'hydroquinone; on peut citer à titre non limitatif l'oxyde d'éthyle, l'oxyde de n-propyle, l'oxyde d'isopropyle, l'oxyde de n-butyle, le dioxane, le diglyme (éther diméthylique du diéthylèneglycol). Parmi les solvants de l'hydroquinone, inertes dans les conditions de la réaction, on fait appel de préférence à ceux qui sont miscibles avec la solution aqueuse d'acide chlorhydrique à la concentration choisie, dans les conditions de température retenues et qui, de ce fait, sont susceptibles d'assurer une miscibilité au moins partielle pendant la réaction de la solution aqueuse d'acide chlorhydrique et de la solution organique d'hydroquinone et par conséquent le meilleur contact possible entre le chlore naissant et l'hydroquinone. De ce point de vue l'oxyde d'éthyle, l'oxyde d'isopropyle, le dioxane et le diglyme sont préférés. L'oxyde d'isopropyle convient tout spécialement bien. La miscibilité de la solution organique d'hydroquinone avec la solution aqueuse d'acide chlorhydrique ne dépend pas seulement de la nature du solvant mais aussi de la concentration de la solution aqueuse d'acide chlorhydrique et de la température. Le choix d'un solvant de l'hydroquinone miscible, dans les conditions de la réaction, à la solution aqueuse d'acide chlorhydrique permet de conduire la chloration préférentiellement en phase homogène.

On a constaté en effet de manière inattendue au vu de l'enseignement de l'état de la technique que les meilleurs résultats sont obtenus lorsque le mélange réactionnel hydroquinone/acide chlorhydrique/eau/solvant est homogène, c'est-à-dire ne forme qu'une seule phase liquide. Dans ces conditions il est possible d'obtenir d'excellents rendements en monochlorohydroquinone en limitant la formation des dichlorohydroquinones et éventuellement l'oxydation de l'hydroquinone par le chlore naissant et/ou par l'eau oxygénée que l'on relève dans les conditions préconisées par le brevet anglais No. 563 541 et la demande de brevet français No. 2 187 735 précités.

La présente invention a donc encore plus spécifiquement pour objet un procédé de chloration de l'hydroquinone essentiellement en monochlorohydroquinone au moyen de chlore à l'état naissant caractérisé en ce que l'on fait réagir l'hydroquinone avec une solution aqueuse d'acide chlorhydrique et de l'eau oxygénée en présence d'un solvant organique de l'hydroquinone inerte selon la revendication 1, miscible, dans les conditions de la réaction, avec la solution aqueuse d'acide chlorhydrique de concentration choisie.

Plus préférentiellement encore la présente invention a pour objet un procédé amélioré de chloration de l'hydroquinone essentiellement en monochlorohydroquinone au moyen de chlore à l'état naissant selon lequel on fait réagir l'hydroquinone avec une solution aqueuse d'acide chlorhydrique et de l'eau oxygénée en présence d'un solvant inerte de l'hydroquinone miscible à la solution aqueuse d'acide chlorhydrique en maintenant le milieu réactionnel homogène pendant la durée de la réaction.

Bien que la concentration de la solution aqueuse d'acide chlorhydrique puisse varier dans de larges limites, elle est choisie, pour un solvant donné, de façon à assurer de préférence la plus grande miscibilité possible du solvant avec la solution chlorhydrique aqueuse dans les conditions de température retenues; en définitive cette concentration, exprimée en moles d'acide chlorhydrique par litre de solution aqueuse, dépend, pour une température donnée, dans une certaine mesure de la nature du solvant choisi. On fait appel de préférence à des solutions aqueuses d'acide chlorhydrique de concentration égale à au moins 7 moles par litre et de préférence à au moins 6 moles par litre. Il n'y a pas de limite supérieure critique de la concentration de la solution chlorhydrique. En pratique il n'y a cependant pas lieu de dépasser une concentration de 12 moles par litre; une concentration de 9 à 11 moles de HCl par litre de solution convient bien.

La quantité d'acide chlorhydrique exprimée en moles de HCl par mole d'hydroquinone n'est pas critique dès lors qu'elle est au moins voisine de la quantité stoechiométrique nécessaire au déroulement de la réaction, c'est-à-dire voisine de 1 mole par mole d'hydroquinone. Le recours à une quantité d'acide chlorhydrique inférieure à 1 mole par mole d'hydroquinone se traduit par une transformation incomplète de l'hydroquinone et ne revêt pas d'intérêt particulier. De préférence la quantité d'acide chlorhydrique est d'au moins 0,9 mole par mole d'hydroquinone; bien qu'il n'y ait pas de valeur supérieure critique il est sans intérêt de mettre en oeuvre plus de 50 moles de HCl par mole d'hydroquinone.

Les quantités relatives de solvant et de solution aqueuse d'acide chlorhydrique ne sont pas critiques. Lorsque, selon un mode préféré de réalisation de l'invention, on conduit le procédé en phase homygène on les choisit de façon à assurer une bonne miscibilité de la solution organique d'hydroquinone et de la solution aqueuse d'acide chlorhydrique; les quantités convenant à l'obtention de cet objectif dépendent de la nature du solvant, de la concentration de la solution aqueuse d'acide chlorhydrique et de la température et doivent donc être déterminées dans chaque cas particulier pour assurer l'homogénéïté du milieu réactionnel pendant la plus grande partie du déroulement de la réaction.

La quantité d'eau oxygénée exprimée en moles de $H_2O_2$ par mole d'hydroquinone est de préférence voisine de la quantité stoechiométrique, c'est-à-dire voisine de 1 mole par mole. En pratique la quantité d'eau

3

oxygénée est comprise entre 0,9 et 1,2 mole de $H_2O_2$ par mole d'hydroquinone et de préférence entre 1 et 1,1 mole par mole d'hydroquinone.

Bien que la concentration de la solution aqueuse d'eau oxygénée exprimée en poids de $H_2O_2$ pour 100 g de solution ne présente aucun caractère critique, il est préférable d'avoir recours à des solutions de concentration suffisante pour ne pas augmenter exagérément le volume de la masse réactionnelle. D'autre part l'emploi de solutions diluées d'eau oxygénée contribue à abaisser, au fur et à mesure du déroulement de la réaction, la concentration de la solution aqueuse d'acide chlorhydrique en raison de l'eau apportée qui s'ajoute à l'eau formée lors de l'oxydation de l'hydracide. Lorsque l'on désire maintenir l'homogénéité du milieu le plus longtemps possible il est préférable de faire appel à des solutions aqueuses d'eau oxygénée de concentration comprises dans un intervalle de 20 à 90 % en poids.

La température à laquelle est réalisée la chloration selon la présente invention peut varier dans de larges limites. En pratique une température comprise entre 0 et 150°C et de préférence entre 10 et 100°C convient bien. La réaction peut être conduite à la pression atmosphérique ou à pression plus élevée si cela s'avère nécessaire, par exemple à la pression autogène du milieu réactionnel.

Le procédé selon l'invention est particulièrement simple à mettre en oeuvre; il suffit en effet d'ajouter progressivement sous agitation la solution d'eau oxygénée dans un récipient réactionnel contenant la solution aqueuse d'acide chlorhydrique et la solution organique d'hydroquinone. Lorsque, selon un mode préféré de réalisation de l'invention, on désire opérer en phase homogène les conditions de réaction sont choisies de façon à ce que le milieu réactionnel reste homogène de préférence sensiblement jusqu'à la fin de l'addition de l'eau oxygénée. Lorsque cette addition est terminée, il est possible suivant le cas de maintenir le milieu réactionnel sous agitation et à la température choisie pour une durée à déterminer dans chaque cas.

Lorsque la réaction est terminée le mélange réactionnel est traité pour récupérer la monochlorhydroquinone. Quand on a opéré en phase homogène il suffit d'ajouter au mélange réactionnel une quantité d'eau destinée à provoquer la démixion d'une phase aqueuse acide et d'une phase organique contenant la monochlorohydroquinone, les dichlorohydroquinones et le cas échéant l'hydroquinone non transformée et on sépare ensuite les phases aqueuse et organique par décantation. La phase aqueuse est lavée à l'aide d'un solvant pour en extraire les chlorohydroquinones et l'hydroquinone. On choisit en général le solvant utilisé pendant la réaction. L'extrait et la phase organique sont réunis, le solvant chassé; les chlorohydroquinones et l'hydroquinone sont séparées et récupérées par les moyens usuels. Lorsque la réaction s'est déroulée en phase hétérogène on sépare les phases aqueuses et organiques par décantation puis traite la phase aqueuse comme précédemment.

Le procédé selon l'invention se prête particulièrement bien à une mise en oeuvre en continu.

Les exemples suivants illustrent l'invention et montrent comment elle peut être mise en pratique.

## Exemple 1

Dans un tricol de 250 ml, muni d'une ampoule de coulée, d'un réfrigérant et d'un agitateur, on introduit:
- hydroquinone: 11 g - 0,1 mole - qualité photographique
- acide chlorhydrique 10N: 80 ml - 0,8 mole.

On rajoute ensuite lentememt sous agitation 40 ml d'éther diisopropylique pur. Il y a dissolution de l'hydroquinone et formation d'une solution homogène à température ambiante.

On coule ensuite lentement, tout en maintenant la température à 20-23°C, 11,42 g d'eau oxygénée à 29,8 % soit 0,1 mole. La durée de la coulée est de 1 h 20 mn et le milieu réactionnel devient hétérogène à la fin de la coulée. On maintient le mélange sous agitation, à température ambiante, pendant 30 minutes, puis l'ensemble est passé en ampoule à décanter. On sépare les deux phases, récupère la phase organique et extrait la phase aqueuse par quatre fois 50 ml d'éther diisopropylique. Les phases organiques sont rassemblées puis on évapore l'éther diisopropylique à l'aide d'un évaporateur rotatif. On obtient un produit solide blanc poudreux dont le poids est de 14,82 g.

Le précipité, analysé par chromatographie en phase liquide haute pression, contient les produits suivants:

| | |
|---|---|
| - hydroquinone | 0,097 g - 0,00825 mole |
| - monochlorohydroquinone | 12,11 g - 0,08382 mole |
| - dichloro-2,5 hydroquinone | 0,847 g - 0,00473 mole |
| - dichloro-2,3 hydroquinone | 0,3 g - 0,00168 mole |

ce qui correspond aux résultats suivants:

| | |
|---|---|
| - taux de transformation de l'hydroquinone | 91,75 % |
| - rendement de monochlorohydroquinone par rapport à l'hydroquinone transformée | 91,35 % |
| - rendement en dichloro-2,5 hydroquinone par rapport à l'hydroquinone transformée | 5,15 % |
| - rendement en dichloro-2,3 hydroquinone par rapport à l'hydroquinone transformée | 1,85 %. |

On charge le produit solide précédent (14,82 g) dans 75 ml d'anhydride acétique pur. On additionne 30 mg d'acide paratoluène sulfonique.

On porte le mélange à 100°C pendant 3 heures, durée au bout de laquelle on ne décèle plus de composés portant un groupement phénolique libre (analyse par chromatographie en couche mince).

La solution est dosée par chromatographie en phase liquide haute pression. On trouve la composition suivante:

- diacétate d'hydroquinone      1,615 g 0,00835 mole
- diacétate de monochlorohydroquinone      19,10 g 0,08364 mole
- diacétate de dichloro-2,5 hydroquinone      1,141 g 0,00433 mole
- diacétate de dichloro-2,3 hydroquinone      0,400 g 0,00152 mole.

Ce qui correspond aux résultats suivants rapportés à l'hydroquinone initialement chargée à la chloration:

- taux de transformation de l'hydroquinone      91,67 %
- rendement en diacétate de monochlorohydroquinone par rapport à l'hydroquinone transformée      91,24 %
- rendement en diacétate de dichloro-2,5 hydroquinone par rapport à l'hydroquinone transformée      4,7 %
- rendement en diacétate de dichloro-2,3 hydroquinone par rapport à l'hydroquinone transformée      1,7 %.

## Exemple 2

Dans un tricol de 250 ml muni d'une ampoule de coulée, d'un réfrigérant et d'un agitateur, on introduit:
- hydroquinone: 11 g (0,1 mole) qualité technique
- acide chlorhydrique 10N: 120 ml (1,2 mole)
- éther diisopropylique pur: 40 ml.

Le mélange est homogène à température ambiante.

On additionne alors, en maintenant la solution entre 22 et 25°C, 12,1 g d'eau oxygénée à 29,5 % soit 0,105 mole. La durée de la coulée est de 1 h 05 mn.

A la fin de la coulée d'eau oxygénée le milieu réactionnel devient hétérogène. On maintient l'agitation pendant 1 h environ. La masse réactionnelle est ensuite introduite dans une ampoule à décanter. On effectue la séparation, récupère la phase organique, lave la phase aqueuse par 4 fois 50 ml d'éther diisopropylique pur.

On rassemble les phases organiques que l'on soumet à l'analyse par chromatographie en phase liquide haute pression. On trouve:

- hydroquinone      0,465 g 0,00423 mole
- monochlorohydroquinone      12,2 g 0,08445 mole
- dichloro-2,5 hydroquinone      1,05 g 0,0059 mole
- dichloro-2,3 hydroquinone      0,37 g 0,0021 mole

ce qui correspond aux résultats suivants:
- taux de transformation de l'hydroquinone      95,8 %
- rendement en monochlorohydroquinone par rapport à l'hydroquinone transformée      88,2 %
- rendement en dichloro-2,5 hydroquinone par rapport à l'hydroquinone transformée      6,15 %
- rendement en dichloro-2,3 hydroquinone par rapport à l'hydroquinone transformée      2,2 %.

## Exemples 3 à 9

On a procédé à la chloration, selon le mode opératoire décrit précédemment, de l'hydroquinone technique utilisée à l'exemple 2 en faisant varier les conditions de la réaction. Des conditions et les résultats obtenus figurent dans le tableau ci-après.

| Exemples | Acide chlorhydrique | | Solvant | | $H_2O_2$ | T en | TT HQ | RT CPHQ | RT DCTHQ |
| | Concentration | Volume | Nature | Volume | en mole | °C | (1) % | (2) % | (3) % |
|---|---|---|---|---|---|---|---|---|---|
| 3 | 10N | 80 ml | OI (4) | 40 ml | 0,105 | 23 | 93,75 | 86,7 | 7,5 |
| 4 | d° | 160 ml | d° | 80 ml | d° | d° | 94,6 | 85,6 | 9,5 |
| 5 | d° | 80 ml | OB (5) | 80 ml | 0,1 | 20 | 99,6 | 78,5 | 11,2 |
| 6 | d° | d° | OE (6) | 40 ml | 0,105 | d° | 84,5 | 86,38 | 10,05 |
| 7 | d° | d° | dioxane | 60 ml | d° | 28 | 91,75 | 86,7 | 10,92 |
| 8 | d° | d° | diglyme | 40 ml | d° | 23 | 90,25 | 84,9 | 9,87 |
| 9 | d° | 50 ml | acide acétique | 50 ml | 0,25 (7) | 100 | 86,7 | 76,5 | 6,39 |

(1) Taux de transformation de l'hydroquinone
(2) Rendment en monochlorohydroquinone par rapport à l'hydroquinone transformée
(3) Rendement en dichlorohydroquinones par rapport à l'hydroquinone transformée
(4) Oxyde d'isopropyle
(5) Oxyde de butyle
(6) Oxyde d'éthyle.
(7) on a chargé 0,25 mole d'hydroquinone.

## Revendications

1. Procédé de chloration d'hydroquinone essentiellement en monochlorohydroquinone au moyen de chlore à l'état naissant caractérisé en ce que l'on fait réagir l'hydroquinone avec une solution aqueuse d'acide chlorhydrique et de l'eau oxygénée en présence d'un solvant organique de l'hydroquinone inerte dans les conditions de la réaction, choisi dans le groupe formé par les acides alcanoïques et les éthers.

2. Procédé selon la revendication 1, caractérisé en ce que le solvant de l'hydroquinone est miscible avec la solution aqueuse d'acide chlorhydrique dans les conditions de la réaction.

3. Procédé selon la revendication 2, caractérisé en ce que la réaction est conduite en phase homogène.

4. Procédé selon l'une quelconque des revendications 1 à 3, caractérisé en ce que le solvant est choisi dans le groupe formé par l'oxyde d'éthyle, l'oxyde d'isopropyle, l'oxyde de n-butyle, le dioxane, le diglyme, l'acide acétique.

5. Procédé selon l'une quelconque des revendications 1 à 4, caractérisé en ce que la concentration de la solution aqueuse d'acide chlorhydrique est au moins égale à 7 moles par litre.

6. Procédé selon l'une quelconque des revendications 1 à 5, caractérisé en ce que la température est comprise entre 0 et 150° C.

7. Procédé selon l'une quelconque des revendications 1 à 6, caractérisé en ce que la quantité d'acide chlorhydrique est d'au moins 1 mole par mole d'hydroquinone.

8. Procédé selon l'une quelconque des revendications 1 à 7, caractérisé en ce que la quantité d'eau oxygénée est comprise entre 1 et 1,2 mole par mole d'hydroquinone.

9. Procédé de chloration de l'hydroquinone selon l'une quelconque des revendications 1 à 8, caractérisé en ce que l'on ajoute progressivement une solution aqueuse d'eau oxygénée sous agitation à un mélange homogène d'une solution aqueuse d'acide chlorhydrique et d'une solution d'hydroquinone dans un solvant organique inerte dans des conditions choisies pour assurer l'homogénéité du milieu réactionnel au moins jusqu'à la fin de l'addition de l'eau oxygénée.

## Patentansprüche

1. Verfahren zur Chlorierung von Hydrochinon im wesentlichen zu Monochlorhydrochinon mittels Chlor in naszierendem Zustand, dadurch gekennzeichnet, daß man das Hydrochinon mit einer wäßrigen Salzsäurelösung und Wasserstoffperoxid in Anwesenheit eines organischen Lösungsmittels für das Hydrochinon, das unter den Reaktionsbedingungen inert ist, und ausgewählt ist aus der Gruppe, gebildet durch die Alkansäuren und Ether, zur Reaktion bringt.

2. Verfahren gemäß Anspruch 1, dadurch gekennzeichnet, daß das Lösungsmittel des Hydrochinons mit der wäßrigen Salzsäurelösung unter den Reaktionsbedingungen mischbar ist.

3. Verfahren gemäß Anspruch 2, dadurch gekennzeichnet, daß die Reaktion in homogener Phase durchgeführt wird.

4. Verfahren gemäß einem der Ansprüche 1 bis 3, dadurch gekennzeichnet, daß das Lösungsmittel ausgewählt ist aus der Gruppe, gebildet durch Ethylether, Isopropylether, n-Butylether, Dioxan, Diglyme, Essigsäure.

5. Verfahren gemäß einem der Ansprüche 1 bis 4, dadurch gekennzeichnet, daß die Konzentration der

6

wäßrigen Salzsäurelösung mindest gleich 7 Mol pro Liter ist.

6. Verfahren gemäß einem der Ansprüche 1 bis 5, dadurch gekennzeichnet, daß die Temperatur zwischen 0 und 150°C liegt.

7. Verfahren gemäß einem der Ansprüche 1 bis 6, dadurch gekennzeichnet, daß die Salzsäuremenge mindestens 1 Mol pro Mol Hydrochinon beträgt.

8. Verfahren gemäß einem der Ansprüche 1 bis 7, dadurch gekennzeichnet, daß die Wasserstoffperoxidmenge zwischen 1 und 1,2 Mol pro Mol Hydrochinon liegt.

9. Verfahren zur Chlorierung von Hydrochinon gemäß einem der Ansprüche 1 bis 8, dadurch gekennzeichnet, daß man eine wäßrige Wasserstoffperoxidlösung unter Rühren fortschreitend zu einem homogenen Gemisch einer wäßrigen Salzsäurelösung und einer Hydrochinonlösung in einem inerten organischen Lösungsmittel zufügt, unter Bedingungen, die ausgewählt sind, um die Homogenität des Reaktionsmilieus mindestens bis zur Beendigung der Zugabe des Wasserstoffperoxids zu sichern.

## Claims

1. Process for the chlorination of hydroquinone principally to monochlorohydroquinone using chlorine in the nascent state, characterized in that hydroquinone is reacted with an aqueous solution of hydrochloric acid and hydrogen peroxide in the presence of an organic solvent for hydroquinone which is inert under the conditions of the reaction, chosen from the group consisting of alkanoic acids and ethers.

2. Process according to Claim 1, characterized in that the solvent for hydroquinone is miscible with the aqueous hydrochloric acid solution under the conditions of the reaction.

3. Process according to Claim 2, characterized in that the reaction is performed in a homogeneous phase.

4. Process according to any one of Claims 1 to 3, characterized in that the solvent is chosen from the group consisting of ethyl ether, isopropyl ether, n-butyl ether, dioxane, diglyme and acetic acid.

5. Process according to any one of Claims 1 to 4, characterized in that the concentration of the aqueous hydrochloric acid solution is at least equal to 7 moles per litre.

6. Process according to any one of Claims 1 to 5, characterized in that the temperature is between 0 and 150°C.

7. Process according to any one of Claims 1 to 6, characterized in that the amount of hydrochloric acid is at least 1 mole per mole of hydroquinone.

8. Process according to any one of Claims 1 to 7, characterized in that the amount of hydrogen peroxide is between 1 and 1.2 moles per mole of hydroquinone.

9. Process for chlorination of hydroquinone according to any one of Claims 1 to 8, characterized in that an aqueous hydrogen peroxide solution is added gradually with stirring to a homogeneous mixture of an aqueous hydrochloric acid solution and a solution of hydroquinone in an organic solvent which is inert under conditions chosen to provide for the homogeneity of the reaction medium at least until the addition of hydrogen peroxide is completed.